# EUROPÄISCHE PATENTSCHRIFT

(11) **EP 0 281 978 B1**
(45) Veröffentlichungstag und Bekanntmachung des Hinweises auf die Patenterteilung: **28.04.1993**
(21) Anmeldenummer: 88103458.1
(22) Anmeldetag: 05.03.1988
(51) Int. Cl.: C12N 7/06

(54) **Inaktivierung von Human Immunodeficiency Virus (HIV) in proteinhaltigen Lösungen durch Phenole**
Inactivation of human immunodeficiency virus (HIV) in proteinic solutions by means of phenol
Inactivation du virus d'immuno-déficience humaine (VIH) dans des solutions protéiniques à l'aide de phénol

(30) Priorität: 12.03.1987 DE 3707987
(43) Veröffentlichungstag der Anmeldung: 14.09.1988
(73) Patentinhaber: BEHRINGWERKE Aktiengesellschaft, 35001 Marburg (DE)
(72) Erfinder: Hilfenhaus, Joachim, Dr., D-3550 Marburg (DE)

(56) Entgegenhaltungen:
- EP-A- 0 197 554
- WO-A-88/09655
- GB-A- 605 687
- US-A- 4 833 165
- AUSTRALIAN DENTAL JOURNAL, Band 32, Nr. 5, 1987, Seiten 368-374; J.A. COTTONE et al.: "Selection for dental practice of chemical disinfectants and sterilants for hepatitis and AIDS"
- LUCRARILE INSTITUTULUI DE CERCETARI VETERINARE SI BIOPREPARATE PASTEUR, Band 16, 1982, Seiten 69-75; N. AVRAM et al.: "Rezistenta virusului leucozei bovine (VLB) la unii factori fizici si chimici"

## Beschreibung

Die Erfindung betrifft ein Verfahren zur Inaktivierung von Human Immunodeficiency Virus (HIV) in proteinhaltigen Lösungen durch die Einwirkung eines Phenols. Auf diese Weise behandelte Präparationen bergen nicht die Gefahr einer AIDS-Übertragung.

Neben anderen Ursachen stellen auch menschliche Blutspenden und Patienten zugeführte humane Plasmaproteine eine potentielle Übertragungsquelle von AIDS dar. Die dabei übertragenen AIDS-Erreger sind Retroviren, deren zuerst aufgefundene Vertreter unter den Bezeichnungen LAV, HTLV-III oder ARV charakterisiert wurden und auf Grund der neuesten Nomenklatur als "Human Immunodeficiency Virus (HIV)" bezeichnet werden. Wie man jetzt weiß, gibt es mehrere HIV-Typen und im Rahmen der Erfindung sollen unter HIV alle zur Gruppe dieser Viren gehörenden Viren verstanden werden. Während die Übertragung von HIV durch humanes Vollblut ebenso wie durch nicht speziell behandelte Faktor VIII-Konzentrate belegt werden konnte, ist eine HIV-Übertragung durch humane Immunglobuline bisher nicht bewiesen.

Unabhängig davon ist es jedoch wünschenswert, bei der Präparation von humanen Plasmaproteinen für die Therapie menschlicher Patienten generell auch Verfahrensschritte anzuwenden, die zu einer Inaktivierung von HIV führen. Der Einsatz HIV-inaktivierender Verfahrensschritte bleibt auch bei routinemäßigem Prüfen von Blutspenden auf Antikörper gegen HIV und die ausschließliche Verwendung anti-HIV-negativer Spenden wünschenswert, um so auch ein potentielles Restrisiko einer AIDS-Übertragung durch die Gabe von Humanplasmaprotein-Präparaten auszuschließen. Die Anwendung jedes möglichen HIV-inaktivierenden Schrittes bei der Herstellung von humanen Immunglobulinen ist zusätzlich zu einer üblichen Alkoholfällung erstrebenswert.

Eine solche HIV-inaktivierende Maßnahme muß HIV eindeutig und auch bei Vorliegen hoher Konzentrationen inaktivieren und die biologische Aktivität und Spezifität der zu behandelnden Plasmaproteine erhalten. Das Verfahren sollte einfach und bezogen auf die Aktivität der Plasmaproteine möglichst verlustlos durchführbar sein. Aus Lucrarile Institutului de Cercetari Veterinare si Biopreparate "Pasteur" 16, Seiten 69 bis 75 (1982) ist bekannt, daß das bovine Leukosevirus, ein Retrovirus, durch eine fünfzehntägige Inkubation in einer 0,5 % Phenol enthaltenden Lösung inaktiviert werden kann.

Es wurde nun gefunden, daß HIV in wässrigen Medien durch Phenol und zwar durch geringe für die Aktivität beispielsweise der Immunglobuline unschädliche Konzentrationen inaktiviert werden können.

Gegenstand der Erfindung ist deshalb ein Verfahren zur Inaktivierung von Human Immunodeficiency Virus (HIV) in Proteinlösungen, dadurch gekennzeichnet, daß einer solchen Lösung ein Phenol in einer Konzentration bis zu 1 g/100 ml Lösung zugesetzt und bei einem pH von 3,5-4,5 bis zu 24 Stunden einwirken gelassen wird.

Als "Phenol" wird eine organisch-chemische Verbindung verstanden, worin mindestens eine Hydroxygruppe unmittelbar an ein aromatisches Ringsystem gebunden ist, wobei weitere Substituenten vorhanden sein können wie beispielsweise in Kresolen.

Bevorzugt wird Phenol selbst verwendet.

Es ist vorteilhaft bei Raumtemperatur und einem pH von 3,5 - 4,5 oder erhöhter Temperatur zu inkubieren. Inkubationszeiten von 0,5 bis 24 Stunden sind zweckmäßig. Es wird solange inkubiert, bis die Lösung nicht mehr infektiös ist.

Proteinlösungen, auf die das beschriebene Verfahren angewandt werden kann, sind beispielsweise Immunglobulinlösungen aber auch Lösungen von monoklonalen Antikörpern, aus natürlichem Material oder gentechnisch gewonnene "Plasmaproteine" wie F VIII, t-PA, Urokinase, Prourokinase, F XIII und AT III, Überstände von Zellkulturen oder Ascitesflüssigkeit von Mäusen. Die Verfahrensbedingungen müssen derart sein, daß die Eigenschaften der Proteine nicht verändert werden. Da die angegebene Menge an Phenol ohne Schwierigkeiten durch Standardmethoden auch aus Präparationen von Plasmaproteinen entfernt werden können, lassen sich nach dem erfindungsgemäßen Verfahren wirtschaftlich retrovirusfreie und damit AIDS-sichere Plasmaproteininpräparate für die Anwendung am Menschen gewinnen.

Nach dem erfindungsgemäßen Verfahren erhaltene Produkte können ebenso für diagnostische Zwecke verwendet werden.

### Beispiele

### HIV-Inaktivierung bei erhöhter Temperatur

Einer humanen Immunglobulinpräparation, die frei von Antikörpern gegen HIV war, wurde infektiöses HIV zugesetzt. Der Virustiter dieser Mischung betrug 7.5 log₁₀-ID₅₀/ml. Anschließend wurde der pH-Wert auf 4 eingestellt und Phenol zugesetzt, so daß die Konzentration nach Durchmischen 0,3 g/100 ml war. Nach 30minütigem Stehen bei Raumtemperatur wurde eine Probe zur Bestimmung des Virus-titers entnommen, der dann bereits auf weniger als 3.5 log₁₀ID₅₀/ml abgefallen war. Die phenolhaltige VirusImmunglobulin-Mischung wurde anschließend 20 Stunden lang bei 40° C im Wasserbad inkubiert. Dann wurde auf infektiöses HIV getestet. 1 ml-Proben waren frei von infektiösem HIV. Dies bedeutet, daß nach dreißigminütiger Inkubation unter den angegebenen Bedingungen eine Inaktivierungsrate von HIV von mehr als 4.0 log₁₀ ID₅₀ und nach weiterer zwanzigstündiger Inkubation bei 40° C eine HIV-Inaktivierungsrate von insgesamt mehr als 7.5 log₁₀ID₅₀ erreicht wurde.

### HIV-Inaktivierung bei Raumtemperatur

Einer von Antikörpern gegen HIV freien humanen Immunglobulinpräparation wurde nach Einstellen auf einen pH-Wert von 4 eine definierte Menge an infektiösem HIV zugesetzt (berechneter Virustiter dieser Mischung 5 log₁₀ID₅₀ /ml) und nach fünfzehnminütigem Stehen bei Raumtemperatur eine Probe zur Bestimmung des Virustiters entnommen. Dieser Titer betrug 2.8 log₁₀ID₅₀/ml. Daraus folgte eine Inaktivierungsratevon 2.2 log₁₀ID₅₀. Anschließend wurde dem Virus-Immunglobulin-Gemisch Phenol in einer Endkonzentration von 0,3 g/100 ml zugesetzt und die resultierende Mischung 30 Minuten bei Raumtemperatur inkubiert. Danach war in 1 ml-Proben dieser Mischung kein infektiöses HIV mehr nachweisbar. Obwohl durch den niedrigen pH-Wert eine teilweise Inaktivierung von HIV erfolgte, war die komplette HIV-Inaktivierung (Inaktivierungsrate von mehr als 10⁵) erst durch den Einsatz von Phenol möglich.

## Patentansprüche

1. Verfahren zur Inaktivierung von Human Immunodeficiency Virus (HIV) in Proteinlösungen, dadurch gekennzeichnet, daß einer solchen Lösung ein Phenol in einer Konzentration von bis zu 0.3 g/100 ml Lösung zugesetzt und bis zu 24 Stunden bei einem pH von 3,5 - 4,5 einwirken gelassen wird.

2. Verfahren nach Anspruch 1, dadurch gekennzeichnet, daß bei einer Temperatur von 15 bis 60°C einwirken gelassen wird, bis keine HIV-Aktivität mehr nachweisbar ist.

3. Verfahren nach Anspruch 1, dadurch gekennzeichnet, daß bei Raumtemperatur einwirken gelassen wird, bis keine HIV-Aktivität mehr nachweisbar ist.

4. Verfahren nach Anspruch 1, dadurch gekennzeichnet, daß bei einer Temperatur von 35 - 45°C einwirken gelassen wird, bis keine HIV-Aktivität mehr nachweisbar ist.

5. Verwendung eines Phenols in einer Konzentration von bis zu 0.3 g/100 ml Lösung und für eine Zeitdauer bis zu 24 Stunden bei einem pH von 3.5 - 4.5 zur Inaktivierung von HIV.

## Claims

1. A process for the inactivation of human immunodeficiency virus (HIV) in protein solutions, which comprises addition of a phenol in a concentration of up to 0.3 g/100 ml of solution to a solution of this type, and allowing it to act for up to 24 hours at a pH of 3.5 - 4.5.

2. The process as claimed in claim 1, wherein it is allowed to act at a temperature of 15 to 60°C until HIV activity is no longer detectable.

3. The process as claimed in claim 1, wherein it is allowed to act at room temperature until HIV activity is no longer detectable.

4. The process as claimed in claim 1, wherein it is allowed to act at a temperature of 35 - 45°C until HIV activity is no longer detectable.

5. The use of a phenol in a concentration of up to 0.3 g/100 ml of solution and for a period of up to 24 hours at a pH of 3.5 - 4.5 for the inactivation of HIV.

## Revendications

1. Procédé pour l'inactivation du virus de l'immunodéficience humaine (VIH) dans des solutions protéiniques, caractérisé en ce que l'on ajoute à une telle solution un phénol à une concentration allant jusqu'à 0,3 g/100 ml de solution, et on laisse agir pendant jusqu'à 24 heures à un pH de 3,5-4,5.

2. Procédé selon la revendication 1, caractérisé en ce que on laisse agir à une température de 15 à 60°C, jusqu'à ce qu'aucune activité de VIH ne soit plus décelable.

3. Procédé selon la revendication 1, caractérisé en ce que on laisse agir à la température ambiante, jusqu'à ce qu'aucune activité de VIH ne soit plus décelable.

4. Procédé selon la revendication 1, caractérisé en ce que on laisse agir à une température de 35 à 45°C, jusqu'à ce qu'aucune activité de VIH ne soit plus décelable.

5. Utilisation d'un phénol à une concentration allant jusqu'à 0,3 g/100 ml de solution, et pendant une durée allant jusqu'à 24 heures, à un pH de 3,5-4,5, pour l'inactivation du VIH.
